(19) Europäisches Patentamt
European Patent Office
Office européen des brevets

(11) **EP 4 267 233 B1**

(12) **EUROPÄISCHE PATENTSCHRIFT**

(45) Veröffentlichungstag und Bekanntmachung des Hinweises auf die Patenterteilung:
**02.10.2024   Patentblatt 2024/40**

(21) Anmeldenummer: **21839455.9**

(22) Anmeldetag: **15.12.2021**

(51) Internationale Patentklassifikation (IPC):
**A61N 1/04** *(2006.01)*      **A61B 5/27** *(2021.01)*
**A61B 5/00** *(2006.01)*      *A61F 13/01* *(2024.01)*

(52) Gemeinsame Patentklassifikation (CPC):
**A61N 1/0492; A61B 5/27; A61B 5/445; A61B 5/6892;** A61B 2562/0209; A61B 2562/125; A61F 13/01029; A61F 13/01042; A61N 1/0484

(86) Internationale Anmeldenummer:
**PCT/EP2021/085807**

(87) Internationale Veröffentlichungsnummer:
**WO 2022/136041 (30.06.2022 Gazette 2022/26)**

(54) **TEXTILES FLACHMATERIAL ZUM ANLEGEN AN EINEN MENSCHLICHEN ODER TIERISCHEN KÖRPER**

FLAT TEXTILE MATERIAL FOR PLACING ON A HUMAN OR ANIMAL BODY

MATÉRIAU TEXTILE PLAT DESTINÉ À ÊTRE PLACÉ SUR UN CORPS HUMAIN OU ANIMAL

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **22.12.2020   DE 102020134698**

(43) Veröffentlichungstag der Anmeldung:
**01.11.2023   Patentblatt 2023/44**

(73) Patentinhaber: **KOB GmbH**
**67752 Wolfstein (DE)**

(72) Erfinder:
• **BORCZYK, Sina**
**67655 Kaiserslautern (DE)**
• **MISHRA, Pulkit**
**04177 Leipzig (DE)**
• **TAMOUÉ, Ferdinand**
**67071 Ludwigshafen am Rhein (DE)**
• **MEYER, Marcin**
**67752 Wolfstein (DE)**

(74) Vertreter: **DREISS Patentanwälte PartG mbB**
**Friedrichstraße 6**
**70174 Stuttgart (DE)**

(56) Entgegenhaltungen:
EP-B1- 3 169 219      DE-A1- 3 213 673
US-A1- 2016 324 439      US-A1- 2019 217 078
US-A1- 2020 297 244

**Beschreibung**

Die Erfindung betrifft ein textiles Flachmaterial gemäß Anspruch 1 zum Anlegen an einen menschlichen oder tierischen Körper zur Erfassung und/oder Übertragung von elektrischen Signalen. Beispielsweise aus DE 10 2017 126 463 A1 ist es bereits bekannt, textilbasierte Kontaktelektroden zum transkutanen Übertragen elektrischer Signale einzusetzen, wobei die Elektrode ein elektrisch leitfähiges Textil und ein elektrisch leitfähiges Polymer aufweist, wobei das elektrisch leitfähige Textil teilweise von dem elektrisch leitfähigen Polymer durchdrungen ist. Die Elektrode ist in einer textilen Flachmaterialschicht gehalten.

Ein weiteres Beispiel von textilbasierten Kontaktelektroden wird in EP 3 169 219 B1 offenbart.

Der vorliegenden Erfindung liegt die Aufgabe zugrunde, ein textiles Flachmaterial der genannten Art bereitzustellen, welches einschließlich der Elektrode textilbasiert, also in einem textilen Verfahren herstellbar ist und welches zur Erfassung und Übertragung von elektrischen Signalen ausgehend vom Körper oder zum Körper verwendet werden kann. Hierunter werden typischerweise auch biophysiologische Potentiale, die am Körper erfasst werden, aber auch den Körper stimulierende Potentiale, die also zum Körper geleitet werden, verstanden.

Vorzugsweise soll das textile Flachmaterial sowohl für die Langzeitverwendung als auch für eine Mehrfachverwendung geeignet sein. Insbesondere könnte das textile Flachmaterial auch zur Unterstützung und Überwachung der Wundbehandlung und Wundversorgung eingesetzt werden, und zwar insbesondere integriert in eine Wundauflage oder einen Wundverband im weit verstandenen Sinn. Das textile Flachmaterial könnte aber auch bei an sich beliebigen physiologischen oder medizinischen Meßvorrichtungen, die zur Verwendung am menschlichen oder tierischen Körper konzipiert sind, verwendet werden.

Zur Lösung dieser Aufgabe wird erfindungsgemäß ein textiles Flachmaterial mit den Merkmalen des Anspruchs 1 vorgeschlagen.

Hierdurch lässt sich einerseits eine in einem textilen Verfahren herstellbare Elektrode aus oder mit elektrisch leitfähigen Fäden herstellen. Insbesondere lässt sich die Elektrode als durch die leitfähigen Fäden ausgebildeter Bereich der ersten Lage ausbilden, welcher zudem weitere Fäden, insbesondere elektrisch nicht leitfähige Fäden, umfassen kann. Dadurch, dass zumindest im Bereich der Elektrode eine körperabgewandte zweite Lage mit elektrisch nicht leitfähigen Fäden mit der ersten relativ geringeren Wasseraufnahmefähigkeit vorhanden ist, lässt sich die Elektrode körperabgewandt durch die zweite Lage abdecken und damit schützen, und es lässt sich durch die erste relativ geringere Wasseraufnahmefähigkeit der Fäden der zweiten Lage, insbesondere durch eine hydrophobe Ausbildung dieser zweiten Lage, ein Schutz vor dem Eindringen von übermäßiger Feuchtigkeit zu der Elektrode als auch ein Verdunstungsschutz, also ein Schutz vor Austrocknen der Körperregion und des textilen Flachmaterials im Bereich der Elektrode, erreichen, so dass deren Funktionsfähigkeit erhalten bleibt und unterstützt wird.

Durch die weiteren Fäden mit der zweiten relativ höheren Wasseraufnahmefähigkeit, die in der ersten Lage oder zwischen der Elektrode und der zweiten Lage in der Längsrichtung verlaufen, können einerseits übermäßige Feuchtigkeit oder gar Flüssigkeit im Elektrodenbereich aufgenommen werden, wobei dies aber nicht zu einer Austrocknung, sondern eher zu einer Feuchtigkeitsspeicherung und -konditionierung des Elektrodenbereichs führt. Dadurch, dass diese Fäden mit der zweiten relativ höheren Wasseraufnahmefähigkeit vorzugsweise nicht in unmittelbarem Hautkontakt sondern möglichst auf der körperabgewandten Seite der Elektrode angeordnet sind, führen sie nicht zur Trockenlegung derjenigen Hautpartie, gegen welche die Elektrode unmittelbar anliegt, sondern es wird im Gegenteil eher davon ausgegangen, dass durch diese Fäden, insbesondere unterstützt durch einen Anpressdruck, eine Feuchtigkeitskonditionierung der Elektrode von der körperabgewandten Seite her erfolgen kann, wodurch ein elektrischer Kontaktwiderstand zwischen der Haut und der Elektrode herabgesetzt werden kann, was zu einer guten Kontaktierung führt.

Die Wasseraufnahmefähigkeit von Fäden kann bestimmt werden entsprechend der Norm DIN 53923:1978-01. Dabei werden anstelle der in der Norm genannten flächigen Probenstücke Fadenabschnitte einer Länge von je 80 mm verwendet. Es werden für jede Messung jeweils mindestens 1 g der Fadenabschnitte als Probe eingesetzt. Alternativ kann auch ein durchgehender Faden, der so lang ist, dass er eine Masse von wenigstens 1g aufweist, verwendet werden. Die an ein Normalklima nach DIN 53802 angeglichene Probe wird auf 0,01 g genau gewogen (Masse trocken mt). Die Fadenabschnitte oder der Faden werden sodann auf einem Drahtnetz nebeneinander liegend oder im Falle des durchgehenden Fadens hin und her gehend angeordnet und an ihren jeweiligen Enden mit Klammern befestigt. Das Drahtnetz mit aufgelegter Probe wird in eine Schale mit destilliertem Wasser von (20+/-1)°C gelegt, so dass die Probe etwa 20 mm unter dem Wasserspiegel ist. Nach einer Einwirkungsdauer von ca 60 Sekunden wird das Drahtnetz mit der Probe entnommen und nach Entfernen einiger Klammern ca. 2 Minuten so gehalten, dass die Fadenabschnitte frei hängend abtropfen können. Die Probe wird dann vom Drahtnetz abgenommen, in ein Wägeglas gelegt und wiederum auf 0,01 g genau gewogen (Masse feucht mf). Die Wasseraufnahmefähigkeit $W_A$ wird nach folgender leicht nachvollziehbarer Rechnung bestimmt und in Gew.-% angegeben:

$$W_A = ((\text{Masse feucht} - \text{Masse trocken}) \, / \, \text{Masse trocken}) * 100$$

$$W_A = ((mf - mt)/mt) *100.$$

Dabei erweist es sich als vorteilhaft, wenn die zweite Wasseraufnahmefähigkeit wenigstens das 1,10-fache, insbesondere wenigstens das 2,0-fache und weiter insbesondere wenigstens das 5,0-fache, insbesondere wenigstens das 10-fache, insbesondere wenigstens das 20-fache der ersten Wasseraufnahmefähigkeit beträgt.

Weiter erweist es sich als vorteilhaft, wenn die erste Wasseraufnahmefähigkeit höchstens 1,0 Gew.-%, insbesondere höchstens 0,1 Gew.-%, insbesondere höchstens 0,01 Gew.-% beträgt. Dies bedeutet, dass die Fadenabschnitte höchstens 1,0 % bzw. höchstens 0,1 % bzw. höchstens 0,01 % ihrer Masse Wasser aufnehmen können.

Ferner erweist es sich als vorteilhaft, wenn die zweite Wasseraufnahmefähigkeit wenigstens 2,0 Gew.-%, insbesondere wenigstens 5,0 Gew.-%, insbesondere wenigstens 20 Gew.-% beträgt. Dies bedeutet dann, dass die Fadenabschnitte wenigstens 2,0 % Prozent ihrer Masse an Wasser aufnehmen können.

Wenn vorliegend von elektrisch leitfähigen Fäden die Rede ist, so bedeutet dies, dass ein solcher elektrisch leitfähiger Faden ein elektrisch leitfähiges Material umfasst, insbesondere damit beschichtet ist, oder aus einem elektrisch leitfähigen Material besteht. Beispielsweise kann ein an sich beliebiger Faden eine metallische und damit elektrisch leitfähige Beschichtung aufweisen, durch welche eine elektrische Leitfähigkeit des Fadens herbeigeführt wird. Ein als elektrisch leitfähig geltender Faden weist dabei einen elektrischen Widerstand von höchstens 2000 Ohm/m, vorzugsweise von höchstens 1000 Ohm/m, bevorzugt von höchstens 500 Ohm/m auf. Dies bedeutet, dass ein Fadenstück von 1 m Länge bei einer an sich üblichen Widerstandsmessung einen elektrischen Widerstand von höchstens 2000 Ohm bzw. höchstens 1000 Ohm bzw. höchstens 500 Ohm aufweist.

Ein elektrisch nicht leitender Faden umfasst in der Regel jedenfalls keine typischen elektrisch leitfähigen Materialien, wie Metalle, und weist insbesondere keine metallische Beschichtung auf. Ein elektrisch nicht leitfähiger Faden weist nach physikalischer Denkgesetzlichkeit einen sehr hohen gegen unendlich strebenden elektrischen Widerstand auf. Ein Faden kann jedenfalls als elektrisch nicht leitfähig gelten, wenn er einen elektrischen Widerstand von mehr als $10^6$ Ohm/m aufweist.

Für die Prüfung, ob ein Faden elektrisch leitfähig oder elektrisch nicht leitfähig ist, werden unter Laborstandardbedingungen konditionierte Fäden verwendet, d.h. Fäden, die trocken sind und die wenigstens 24 Stunden lang bei einer Temperatur von 20°C und einer relativen Luftfeuchtigkeit von 65 % konditioniert wurden.

Das textile Flachmaterial ist in vorteilhafter Weise dadurch ausgebildet, dass die elektrisch leitfähigen Fäden der Elektrode in der Längsrichtung sich erstreckende elektrisch leitfähige Fäden und in der Querrichtung sich erstreckende elektrisch leitfähige Fäden umfassen.

Hierdurch lässt sich die Elektrode in vorteilhafter Weise als ein textiles Gebilde, insbesondere als ein Gewebe herstellen.

Weiter erweist es sich als vorteilhaft, wenn die elektrisch nicht leitfähigen Fäden der zweiten Lage in der Längsrichtung sich erstreckende elektrisch nicht leitfähige Fäden und in der Querrichtung sich erstreckende elektrisch nicht leitfähige Fäden umfassen, die jeweils die erste Wasseraufnahmefähigkeit aufweisen.

Es kann sich insbesondere als vorteilhaft erweisen, wenn die zweite Lage aus elektrisch nicht leitfähigen Fäden besteht, insbesondere aus Fäden, die jeweils die erste Wasseraufnahmefähigkeit aufweisen.

Es kann sich insbesondere als vorteilhaft erweisen, wenn die Elektrode aus elektrisch leitfähigen Fäden besteht. Weiter kann das textile Flachmaterial so ausgebildet sein, dass wenigstens ein an die Elektrode sich in der Querrichtung anschließender Seitenbereich, insbesondere ein an die Elektrode sich in der Querrichtung anschließender erster Seitenbereich und ein an die Elektrode sich in der Querrichtung anschließender zweiter Seitenbereich gegenüberliegend dem ersten Seitenbereich vorhanden ist. Bei dieser Ausführungsform ist die Elektrode des Flachmaterials also in der Querrichtung einseitig oder beidseitig von dem Seitenbereich flankiert. Dieser jeweilige Seitenbereich des Flachmaterials ist von der ersten Lage und/oder von der zweiten Lage gebildet, d.h. die erste Lage und/oder die zweite Lage sind dort flächenhaft erstreckt oder vorhanden.

Nach einer weiteren Ausführungsform des erfindungsgemäßen Flachmaterials ist vorgesehen, dass die Elektrode oder die Elektrode und der oder die Seitenbereiche einen Elektrodenabschnitt des Flachmaterials bilden, und dass sich an den Elektrodenabschnitt in der Längsrichtung ein Zwischenabschnitt des Flachmaterials anschließt.

Weiter kann eine alternierende Ausgestaltung so ausgebildet sein, dass sich ein weiterer Zwischenabschnitt an den Elektrodenabschnitt in der Längsrichtung gegenüber dem Zwischenabschnitt anschließt. Bei dieser Ausführungsform ergibt sich also bei dem textilen Flachmaterial in der Längsrichtung eine Anordnung von Zwischenabschnitt - Elektrodenabschnitt - Zwischenabschnitt.

Es kann sich auch als vorteilhaft erweisen, dass ein weiterer Elektrodenabschnitt sich in der Längsrichtung an den Zwischenabschnitt anschließt, wobei der Zwischenabschnitt dann in der Längsrichtung zwischen den Elektrodenabschnitten angeordnet ist. Bei dieser letzten Ausführungsform ergibt sich also bei dem textilen Flachmaterial in der Längsrichtung eine Anordnung von Elektrodenabschnitt - Zwischenabschnitt - Elektrodenabschnitt.

Es kann sich aber auch als besonders vorteilhaft erweisen, wenn das textile Flachmaterial in der Längsrichtung im Wesentlichen endlos ausgebildet ist, wobei sich jeweils ein Elektrodenabschnitt und ein Zwischenabschnitt abwechseln.

Es erweist sich hinsichtlich einer durchgehend textilen Herstellbarkeit als vorteilhaft, wenn die erste Lage in dem oder den Seitenbereichen des Elektrodenabschnitts in der Längsrichtung sich erstreckende elektrisch nicht leitfähige Fäden und in der Querrichtung sich erstreckende elektrisch leitfähige Fäden umfasst. Dies ermöglicht es, dass die erste Lage in der Querrichtung außerhalb des eigentlichen Elektrodenbereichs zumindest auch von denselben in der Querrichtung sich erstreckenden leitfähigen Fäden gebildet ist, welche im eigentlichen Elektrodenbereich, also in der Querrichtung zwischen den Seitenbereichen, die Elektrode bilden.

Es erweist sich weiter als vorteilhaft, wenn die in der Längsrichtung sich erstreckenden Fäden mit der zweiten relativ höheren Wasseraufnahmefähigkeit in der Querrichtung zwischen dem ersten Seitenbereich und dem zweiten Seitenbereich, angeordnet sind. Dies bedeutet, dass diese Fäden vorzugsweise in der Querrichtung nur im Bereich der Elektrode vorhanden sind, also in der Querrichtung nicht außerhalb des eigentlichen Elektrodenbereichs verlaufen.

In weiterer Ausbildung erweist es sich als vorteilhaft, wenn diese Fäden mit der zweiten relativ höheren Wasseraufnahmefähigkeit flottierend zwischen der Elektrode und der zweiten Lage vorgesehen sind.

Hiermit ist gemeint, dass diese Fäden im Bereich der Elektrode nicht in die erste und die zweite Lage textil eingebunden sind, sondern zwischen diesen Lagen insbesondere im Wesentlichen parallel erstreckt zueinander verlaufen. Auf diese Weise können diese hydrophileren Fäden ihre feuchtigkeitskonditionierende Wirkung unbehindert durch eine textile Bindung entfalten.

Es erweist sich weiter als vorteilhaft, wenn die erste Lage und die zweite Lage zumindest im Elektrodenabschnitt lose aneinander an- oder aufliegen. Auch durch diese weitere Maßnahme wird ein Zwischenraum zwischen der ersten und der zweiten Lage geschaffen, in dem sich die vorgenannten Fäden mit der zweiten Wasseraufnahmefähigkeit erstrecken können und je nach Flüssigkeits- oder Feuchtigkeitsanfall mehr oder weniger Feuchtigkeit aufnehmen und dabei quellen können.

Es kann sich auch als vorteilhaft erweisen, wenn die erste Lage und die zweite Lage im Zwischenabschnitt miteinander verbunden sind.

Insbesondere kann es sich weiter als vorteilhaft erweisen, wenn die erste Lage und die zweite Lage in dem Zwischenabschnitt zur Bildung einer gemeinsamen textilen Trägerlage zusammengeführt sind. Wenn das textile Flachmaterial - wie eingangs erwähnt - in der Längsrichtung aufeinander folgend und insbesondere endlos abwechselnd einen Elektrodenabschnitt und einen Zwischenabschnitt aufweist, so alternieren mehrlagige Elektrodenabschnitte und einlagige Zwischenabschnitte. Dies hat den Vorteil, dass die beiden Lagen im Zwischenabschnitt eine stabile textile Trägerlage ausbilden und dass im Elektrodenabschnitt die erste und die zweite Lage in Dickenrichtung voneinander getrennt und insbesondere aneinander anliegend vorliegen, wie dies vorausgehend auch schon ausgeführt wurde.

In weiterer Fortbildung des erfindungsgemäßen Flachmaterials wird vorgeschlagen, dass die Fäden mit der zweiten Wasseraufnahmefähigkeit in dem Zwischenabschnitt zum Anlegen an den Körper ausgebildet und angeordnet sind.

Dies macht es möglich, dass diese relativ hydrophileren Fäden im Zwischenabschnitt gezielt zur Trockenlegung der Körperoberfläche genutzt werden können. Im Zwischenabschnitt, also in dem Bereich in der Längsrichtung zwischen den Elektroden, ist eine feuchte Körperoberfläche auch unvorteilhaft, weil es messtechnisch erwünscht ist, die Bereiche zwischen zwei Messelektroden möglichst elektrisch voneinander zu isolieren, also einen elektrischen Kurzschluss zwischen den Elektroden durch eine elektrisch leitende Oberflächenverbindung über eine feuchte Körperoberfläche zu verhindern.

Nach einem weiteren Gedanken von besonderer Bedeutung erweist es sich als vorteilhaft, wenn die in der Längsrichtung angeordneten elektrisch leitfähigen Fäden der Elektrode durch die zweite Lage und/oder die Trägerlage in Dickenrichtung hindurchtreten und auf der körperabgewandten Seite der zweiten Lage bzw. der Trägerlage vorzugsweise flottierend angeordnet sind. Wenn diese in der Längsrichtung verlaufenden elektrisch leitfähigen Fäden sich in Fertigungsrichtung des textilen Flachmaterials, also in der Längsrichtung endlos erstrecken, so können sie auch die jeweilige in der Längsrichtung folgende Elektrode bilden. Solchenfalls kann also ein endloses Fertigungsverfahren zur Herstellung des textilen Flachmaterials praktiziert werden.

Es ist nun möglich, diese in der Längsrichtung verlaufenden elektrisch leitfähigen Fäden der Elektrode in dem Bereich zwischen zwei aufeinander folgenden Elektroden zu schneiden und vorzugsweise die jeweiligen Schnittenden zu einem Bündel zusammenzufassen. Hierdurch kann ein elektrischer Anschluss gebildet werden. Es wäre aber auch denkbar, die elektrisch leitfähigen Fäden jeweils insbesondere nahe der Elektrode zu schneiden und ein hierbei entstehendes Zwischenstück zu verwerfen. Sollte es hingegen in einzelnen Anwendungen erwünscht sein, nebeneinander angeordnete Elektroden als Kontaktbereiche elektrisch leitend miteinander verbunden zu halten, so würde man eine Trennung, d.h. Kappung, der Fäden nicht vornehmen.

Nach einer weiteren Ausführungsform erweist es sich als vorteilhaft, dass der Zwischenabschnitt einen Mittenbereich umfasst, wobei der Mittenbereich eine im Wesentlichen der Elektrode entsprechende Quererstreckung in der Querrichtung aufweist, wobei im Mittenbereich in der Längsrichtung sich erstreckende im Wesentlichen parallel nebeneinander angeordnete Fäden mit der zweiten Wasseraufnahmefähigkeit und in der Längsrichtung sich erstreckende und im Wesentlichen parallel nebeneinander angeordnete elektrisch nicht leitfähige Fäden mit der ersten Wasseraufnahmefähigkeit vorgesehen sind.

In der Querrichtung außerhalb eines Mittenbereichs ist der Zwischenabschnitt insbesondere von in der Längsrichtung sich erstreckenden elektrisch nicht leitfähigen Fäden und von in der Querrichtung sich erstreckenden elektrisch leitfähigen und/oder elektrisch nicht leitfähigen Fäden gebildet.

Bei einer weiteren besonders vorteilhaften Ausführungsform ist vorgesehen, dass die erste Lage und die zweite Lage derart angeordnet und miteinander verbunden sind, dass im Elektrodenabschnitt ein sich in der Querrichtung erstreckender Tunnel gebildet ist, der durch die erste und zweite Lage begrenzt wird.

Dies eröffnet die Möglichkeit, diesen Tunnel, also den Zwischenraum zwischen der ersten und der zweiten Lage, nicht nur als Erstreckungsraum für die oben erwähnten Fäden mit der zweiten Wasseraufnahmefähigkeit vorzusehen, sondern einen zusätzlichen Raum für Funktionselemente vorzusehen. Insbesondere ist es denkbar und vorteilhaft, dass in den Tunnel ein Druckelement einsetzbar ist, welches die erste Lage und die Elektrode und gegebenenfalls die quer durch den Tunnel flottierend geführten hydrophileren Fäden mit der zweiten Wasseraufnahmefähigkeit, zum Körper hindrängt. Durch Einsetzen eines zusätzlichen Elements kann somit ein Anpressdruck erzielt werden, welcher die Elektrode gegen die Körperoberfläche drückt, was die Ausbildung eines guten elektrischen Kontakts unterstützt. Der durch den Tunnel gebildete Aufnahmeraum kann aber auch zur Aufnahme elektrischer Komponenten, insbesondere Verstärkerkomponenten oder an sich beliebiger elektrischer oder elektronischer Mess- oder Verstärker- oder Steuereinrichtungen verwendet werden.

Es wird weiter vorgeschlagen, dass die Elektrode zum Körper erhaben gegenüber den Seitenbereichen und/oder gegenüber dem Zwischenabschnitt und/oder gegenüber der Trägerlage ausgebildet ist. Dies kann beispielsweise dadurch realisiert werden, dass im eigentlichen Elektrodenbereich eine größere Anzahl von in der Längsrichtung und/oder in der Querrichtung sich erstreckenden Fäden vorgesehen sind als in den angrenzenden Bereichen außerhalb der Elektrode. Es ist aber auch denkbar, dass dies durch die schon erwähnte abwechselnd mehrlagige und einlagige Ausbildung erreicht werden kann. Wenn vorausgehend von einem textilen Flachmaterial und von in der Längsrichtung und in der Querrichtung verlaufenden Fäden die Rede ist, so ist die spezielle Art der textilen Bindung nicht zwingend vorgegeben, solange sich Fäden im Wesentlichen in der Längsrichtung und quer hierzu erstrecken, was bislang nicht im Sinne einer geometrisch streng linearen Erstreckung gemeint ist. Es wird aber vorzugsweise Schutz beansprucht für ein textiles Flachmaterial, bei dem die erste und/oder die zweite Lage von einem Gewebe gebildet ist, wobei eine Kettrichtung des Gewebes in der Längsrichtung verläuft und eine Schussrichtung des Gewebes in der Querrichtung verläuft. In diesem Fall handelt es sich bei vorausgehend erwähnten in der Längsrichtung erstreckten Fäden um Kettfäden und bei vorausgehend erwähnten in der Querrichtung erstreckten Fäden um Schussfäden des Gewebes. Gegenstand der Erfindung ist auch ein Wundverband zur Unterstützung und Überwachung der Wundbehandlung der ein erfindungsgemäßes textiles Flachmaterial aufweist.

Vorzugsweise kann das gesamte textile Flachmaterial in Form eines von Kettfäden und Schussfäden gebildeten Gewebes ausgebildet sein. Die Kettfäden werden dabei vorzugsweise endlos in einer Produktionsrichtung des textilen Flachmaterials zugeführt. Die hierzu quer erstreckten Schussfäden können an seitlichen Längsrändern des textilen Flachmaterials frei enden, oder es kann dort eine an sich beliebige Randausbildung, insbesondere in Form eines Saums geschaffen werden. Auch können die Querränder oder Längsränder eines Abschnitts des textilen Flachmaterials mit einer an sich beliebigen Randausbildung, insbesondere Besäumung versehen werden.

Weitere Merkmale, Einzelheiten und Vorteile der Erfindung ergeben sich aus den beigefügten Patentansprüchen und aus der zeichnerischen Darstellung und nachfolgenden Beschreibung einer bevorzugten Ausführungsform des erfindungsgemäßen textilen Flachmaterials.

In der Zeichnung zeigt:

Figur 1  eine schematische Draufsicht auf eine hautzugewandte Seite eines erfindungsgemäßen textilen Flachmaterials;

Figur 2  eine schematische Draufsicht auf eine hautabgewandte Seite des Flachmaterials gemäß Fig. 1;

Figur 3  eine schematische dreidimensionale Darstellung eines Abschnitts eines erfindungsgemäßen textilen Flachmaterials mit Blick von oben auf eine körperzugewandte Seite;

Figur 4a  eine weitere Darstellung entsprechend Figur 3; und

Figur 4b  eine schematische Seitenansicht des Abschnitts nach Figur 3;

Figur 5  eine weitere schematische Darstellung des Abschnitts nach Figur 3 mit Blick von unten auf eine körperabgewandte Seite;

Figur 6    eine weitere schematische Darstellung entsprechend Figur 5 mit geschnittenen und gebündelten elektrisch leitfähigen Fäden;

Figur 7    eine Darstellung eines Körperteils mit zwei an das Körperteil angelegten Abschnitten eines erfindungsgemäßen textilen Flachmaterials in einer Betriebssituation; und

Figur 8    eine weitere Darstellung eines Körperteils mit einem ringförmigen geschlossenen Band aus einem erfindungsgemäßen textilen Flachmaterial mit einer Mehrzahl von Elektrodenabschnitten und Zwischenabschnitten.

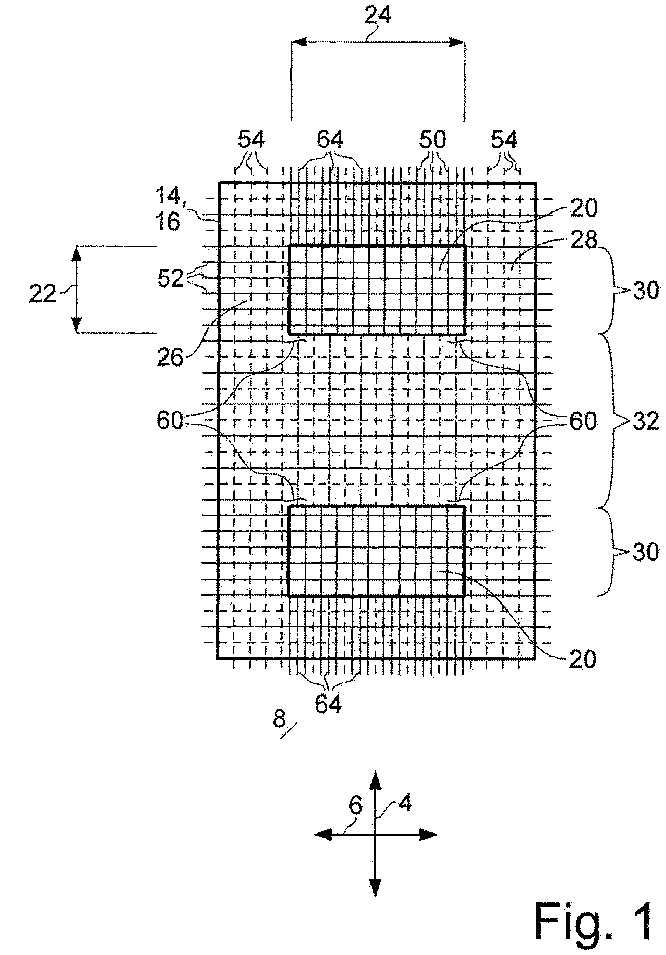

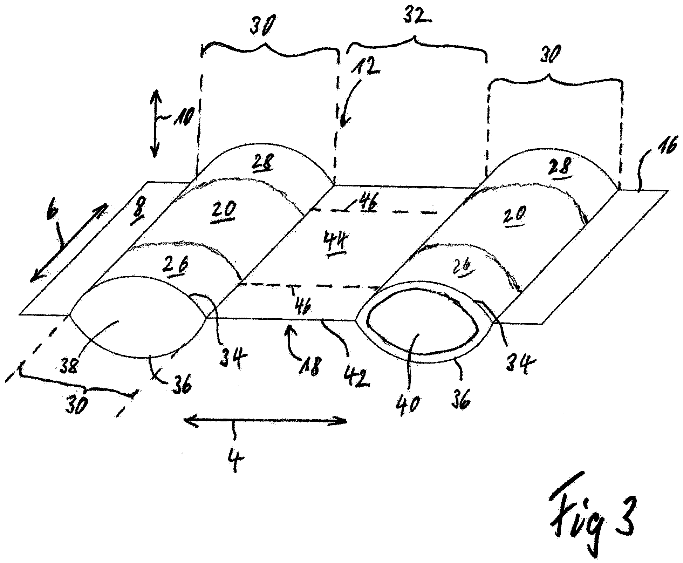

Die Figuren zeigen ein erfindungsgemäßes textiles Flachmaterial 2 und verdeutlichen teils stark schematisch dessen Aufbau. Das Flachmaterial 2 ist dazu vorgesehen, an einen menschlichen oder tierischen Körper angelegt zu werden und elektrische Signale zu erfassen und/oder an den Körper zu übertragen. Das textile Flachmaterial 2 erstreckt sich in einer Längsrichtung 4 und in einer Querrichtung 6 und in einer mit der Zeichnungsebene der Figur 1 übereinstimmenden Erstreckungsebene 8. Ferner sei eine orthogonal zur Erstreckungsebene 8 verlaufende Dickenrichtung 10 eingeführt, die aus Figur 3 ersichtlich ist.

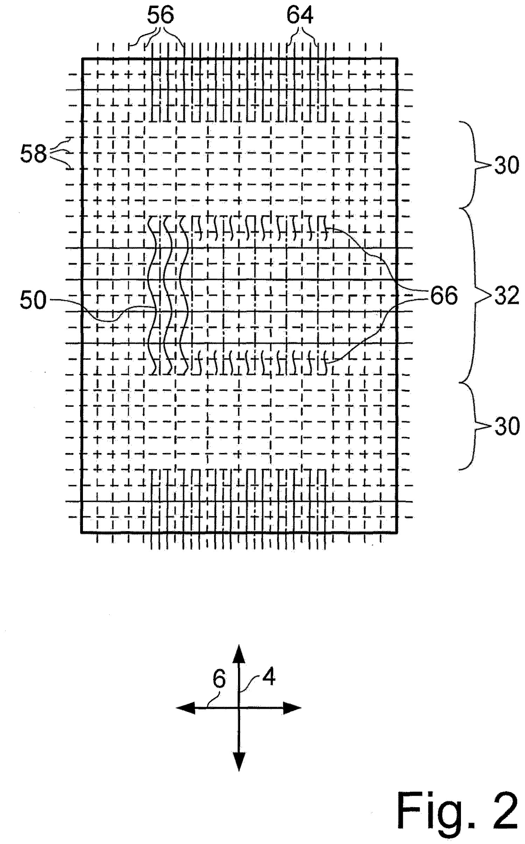

Figur 1 zeigt eine Draufsicht auf eine körperzugewandte Seite 12 eines durch einen rechteckförmigen Rahmen 14 angedeuteten Abschnitts 16 des textilen Flachmaterials 2. Figur 2 zeigt eine Draufsicht auf eine körperabgewandte Seite 18 des Abschnitts 16. In der stark schematischen perspektivischen Darstellung des Flachmaterialabschnitts 16 gemäß Figur 3 ist die körperzugewandte Seite 12 der Blickrichtung zugewandt oben und die körperabgewandte Seite 18 ist der Blickrichtung abgewandt unten.

Der Abschnitt 16 des textilen Flachmaterials 2 umfasst zur Erfassung oder Übertragung von elektrischen Signalen im hier dargestellten Fall beispielhaft zwei Elektroden 20, die beispielhaft rechteckförmig ausgebildet und in Figur 1 mit einem Rahmen 21 angedeutet sind. Sie erstrecken sich über eine Elektrodenlänge 22 in der Längsrichtung 4 und über eine Elektrodenbreite 24 in der Querrichtung 6. In Querrichtung 6 außerhalb der jeweiligen Elektrode 20 schließt sich ein erster und ein zweiter Seitenbereich 26, 28 des Abschnitts 16 an. Jeder Seitenbereich 26, 28 ist auch über die Elektrodenlänge 22 und in Querrichtung 6 jeweils bis zu dem Rand des textilen Flachmaterials 2 erstreckt. Der Bereich der jeweiligen Elektrode 20 bildet zusammen mit dem ersten und dem zweiten Seitenbereich 26, 28 jeweils einen Elektrodenabschnitt 30 des textilen Flachmaterials 2 bzw. des Abschnitts 16. In Längsrichtung 4 zwischen den Elektrodenabschnitten 30 ist ein Zwischenabschnitt 32 des textilen Flachmaterials 2 vorgesehen bzw. angeordnet. In diesem Zwischenabschnitt 32 soll bei den meisten Anwendungen in der Längsrichtung 4 eine weitgehende elektrische Trennung der beiden Elektroden 20 realisiert sein.

Wie noch im Einzelnen dargestellt werden wird, kann das erfindungsgemäße textile Flachmaterial 2 in der Längsrichtung 4 endlos hergestellt werden, so dass aus einem endlos hergestellten Streifen oder Band des erfindungsgemäßen Flachmaterials die in den Figuren angedeuteten Flachmaterialabschnitte 16 als Längsabschnitte dieses endlosen Flachmaterials 2 erhalten werden können. So ist es insbesondere denkbar, dass ein betreffender Abschnitt 16 des Flachmaterials 2 auch mehrere Elektroden 20 aufweisen kann, zwischen denen in der Längsrichtung 4 wie dargestellt Zwischenabschnitte 32 angeordnet sind, welche die Elektroden 20 möglichst elektrisch voneinander trennen oder aber für bestimmte Anwendungen in elektrischer Verbindung zueinander belassen.

Wie man anhand der stark schematisierten Darstellung des Abschnitts 16 des textilen Flachmaterials 2 in der Figur 3 erkennt, umfasst das textile Flachmaterial 2 im jeweiligen Elektrodenabschnitt 30 eine körperzugewandte erste Lage 34 und eine körperabgewandte zweite Lage 36, die im jeweiligen Elektrodenabschnitt 30 im bevorzugt dargestellten Fall insbesondere lose gegeneinander anlegbar sind und/oder, wie in der Figur 3 übertrieben angedeutet, aufweitbar sind und dann einen in der Querrichtung 6 erstreckten Tunnel 38 begrenzen. Der Tunnel 38 kann sich vorzugsweise über die gesamte Quererstreckung des Flachmaterialabschnitts 16 erstrecken, also eine durchgehende lichte Querschnittsfläche begrenzen, in die beispielsweise ein in Figur 3 rechts angedeutetes Druckelement eingesetzt werden kann, um die Elektrode 20 des Flachmaterials 2 in Richtung auf den Körper zu drängen oder zu drücken, um die Erreichung einer guten elektrischen Kontaktierung der Elektrode 20 mit der Körperoberfläche zu unterstützen. Dies ist aber optional, und im Gebrauch kann der Tunnel 38 auch unbefüllt bleiben und die Lagen 34, 36 gewissermaßen lose gegeneinander anliegen. Sie könnten auch erwünschtenfalls in an sich beliebiger Weise, etwa durch Nähte, miteinander verbunden sein.

Wie sich ebenfalls am besten aus Figur 3 ergibt, sind die körperzugewandte erste Lage 34 und die körperabgewandte zweite Lage 36 in dem Zwischenabschnitt 32 zur Bildung einer gemeinsamen textilen Trägerlage 42 zusammengeführt, was nachfolgend noch erläutert werden wird. Beim Übergang des Zwischenabschnitts 32 in den daran anschließenden weiteren Elektrodenabschnitt 30 werden die Fäden der textilen Trägerlage 42 dann wieder in Dickenrichtung 10 auseinandergeführt, um die dortige körperzugewandte erste Lage 34 und körperabgewandte zweite Lage 36 zu bilden.

Schließlich sei noch in dem Zwischenabschnitt 32 ein Mittenbereich 44 definiert, der sich in Querrichtung 6 zwischen zwei gestrichelt angedeuteten Linien 46 befindet und dessen Quererstreckung ungefähr der Elektrodenbreite 24 entspricht.

Es werden nun die Bildung der Lagen, der Elektrode, der Bereiche und Abschnitte des erfindungsgemäßen textilen Flachmaterials 2 erläutert.

Die jeweilige Elektrode 20 umfasst in der Längsrichtung 4 erstreckte elektrisch leitfähige Fäden 50 und in der Querrichtung 6 erstreckte elektrisch leitfähige Fäden 52. Im beispielhaft dargestellten Fall bilden diese elektrisch leitfähigen Fäden 50, 52 sowohl die Elektrode 50 als auch die erste körperzugewandte Lage 34 im Bereich der Elektrode.

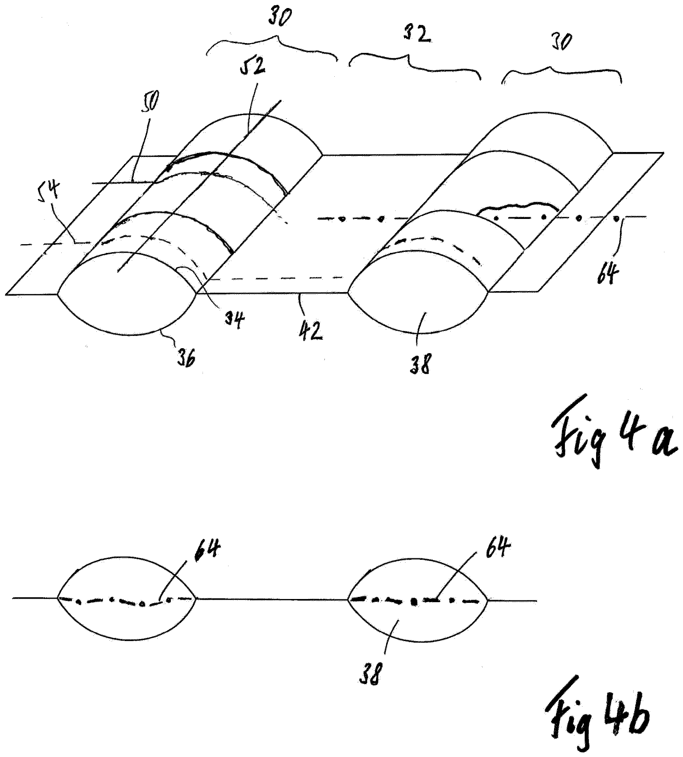

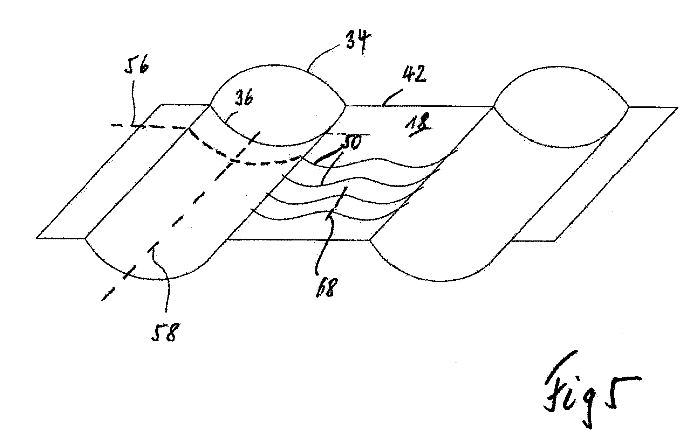

Der Verlauf eines einzigen elektrisch leitfähigen Fadens 50 als Kettfaden und 52 als Schussfaden ist schematisch in Figuren 4 und 5 angedeutet. Dabei zeigt Figur 4 den Flachmaterialabschnitt 16 in der Perspektive und Sichtrichtung von Figur 3, und Figur 5 zeigt den Flachmaterialabschnitt 16 von unten mit Blickrichtung auf die körperabgewandte Seite 18 des Abschnitts 16. In Figur 5 mit Blick auf die körperabgewandte Seite 18 sind indessen mehrere in Längsrichtung 4 verlaufende elektrisch leitfähige Fäden 50 in einer jeweiligen Wellenlinie angedeutet, um einen flottierenden, also nicht gewebten, Verlauf dieser Fäden in dem Zwischenabschnitt 32 zu verdeutlichen. Ein jeweiliger Faden 50 tritt also beim Übergang vom Elektrodenabschnitt 30 in den Zwischenabschnitt 32 in Dickenrichtung 10 durch die körperabgewandte zweite Lage 36 bzw. die Trägerlage 42 im Zwischenabschnitt 32 hindurch und verläuft wie in Figur 5 angedeutet in der Längsrichtung 4 weiter flottierend und gewissermaßen parallel zu der noch zu erläuternden Trägerlage 42.

In Querrichtung 6 außerhalb der Elektrode 20, also in Seitenbereichen 26, 28 des Fachmaterials 2, ist die körperzugewandte erste Lage 34 von in der Längsrichtung 4 verlaufenden elektrisch nicht leitfähigen Fäden 54 und von den auch die Elektrode 20 bildenden und in der Querrichtung 6 verlaufenden elektrisch leitfähigen Fäden 52 gebildet. Es könnten weitere Fäden, insbesondere in der Längsrichtung 6, vorhanden sei, was aber beispielhaft nicht der Fall ist.

Die körperabgewandte zweite Lage 36 ist im beispielhaft dargestellten Fall im Bereich des Elektrodenabschnitts 30 des textilen Flachmaterials 2 von in der Längsrichtung 4 sich erstreckenden elektrisch nicht leitfähigen Fäden 56 und von in der Querrichtung 6 sich erstreckenden elektrisch nicht leitfähigen Fäden 58 gebildet. Dabei bilden die in Längsrichtung erstreckten Fäden 56 als Kettfäden mit den in Querrichtung 6 erstreckten Fäden 58 als Schussfäden ein Gewebe. Auch hier ist zur Veranschaulichung in Figur 5 jeweils ein einziger Faden 56 bzw. 58 andeutet.

Die Fäden 56, 58 weisen eine erste Wasseraufnahmefähigkeit wie eingangs erläutert auf. Die zweite Lage 36 ist daher als eher hydrophob zu bezeichnen, so dass sie einen Zutritt von Flüssigkeit oder übermäßiger Feuchtigkeit von außen durch die zweite Lage 36 hindurch in Richtung auf die jeweilige Elektrode 20 behindert bzw. abhält.

Im beispielhaft dargestellten Fall gehen die körperzugewandte erste Lage 34 und die körperabgewandte zweite Lage 36 im Zwischenabschnitt 32 ineinander über zur Bildung der gemeinsamen textilen Trägerlage 42. Diese Trägerlage 42 umfasst demgemäß im Zwischenabschnitt 32 die in Längsrichtung erstreckten elektrisch leitfähigen Fäden 52 sowie die in Längsrichtung erstreckten elektrisch nicht leitfähigen Fäden 54 sowie die in Querrichtung erstreckten nicht leitfähigen Fäden 58, die nicht nur im Elektrodenabschnitt 30, sondern auch im Zwischenabschnitt 32 vorhanden sind. Wie in Figur 1 am Übergang des Elektrodenabschnitts 30 in den Zwischenabschnitt 32 angedeutet ist, sind dort einige benachbarte in Querrichtung verlaufende elektrisch leitfähige Fäden 52 beidseits geschnitten (s. Bezugszeichen 60), um eine elektrische Isolierung der Elektrode 30 von der textilen Trägerlage 42 im Zwischenabschnitt 32 zu erreichen.

Das textile Flachmaterial 2 umfasst weitere in der Längsrichtung 4 erstreckte Fäden 64, die aber nur in einem mittleren Bereich, welcher ungefähr der Elektrodenbreite 24 entspricht, in der Längsrichtung 4 entlang des gesamten textilen Flachmaterials verlaufen. Diese Fäden 64 verlaufen im Elektrodenabschnitt 30 beispielhaft und bevorzugt flottierend, d.h. sie verlaufen in der Längsrichtung 4 zwischen der körperzugewandten ersten Lage 34 und der körperabgewandten zweiten Lage 36 und durchqueren gewissermaßen den durch diese Lagen gebildeten Tunnel 38. Sie sind dann als weitere Kettfäden in dem Zwischenabschnitt 32, also in der textilen Trägerlage 42, eingewoben. Zur weiteren Anschauung ist ein einziger dieser Fäden 64 in der Figur 4a in der rechten Hälfte und mehrere dieser Fäden 64 in den Figuren 1 und 2 als strichpunktierte Linie dargestellt. Figur 4b verdeutlicht in einer schematischen Seitenansicht den flottierenden Verlauf dieser Fäden 64 durch den Tunnel 38 hindurch.

Diese weiteren in der Längsrichtung verlaufenden Fäden 64 sind elektrisch im Wesentlichen nicht leitend, und sie weisen eine zweite Wasseraufnahmefähigkeit auf, die höher ist als die erste Wasseraufnahmefähigkeit der Fäden 56, 58 der zweiten Lage 36. Die zweite Wasseraufnahmefähigkeit ist vorzugsweise deutlich höher als die erste Wasseraufnahmefähigkeit der nicht leitenden Fäden 56, 58 der zweiten Lage 36. Insbesondere erweist es sich als vorteilhaft, wenn die zweite Wasseraufnahmefähigkeit der Fäden 64 diese Fäden als hydrophil erscheinen lässt und wenn die erste Wasseraufnahmefähigkeit der Fäden 56, 58 der zweiten Lage 36 diese Lage hierdurch als hydrophob erscheinen lässt. Die Wasseraufnahmefähigkeit von Fäden kann wie eingangs schon ausgeführt bestimmt werden.

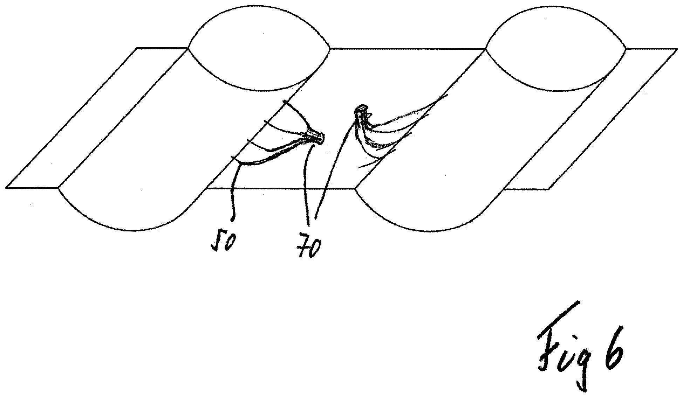

Die eine relativ höhere zweite Wasseraufnahmefähigkeit aufweisenden in Längsrichtung 4 durch das gesamte textile Flachmaterial 2 erstreckten hydrophilen Fäden 64 sind im Zwischenabschnitt 32 bei der textilen Trägerlage 42 in dem eingangs erwähnten Mittenbereich 44 so vorgesehen und geführt, dass sie, z.B. als Kettfäden der textilen Trägerlage 42 immer wieder in unmittelbare Anlage an die Körperoberfläche gelangen. Auf diese Weise können die Wasser und Feuchtigkeit aufnehmenden Fäden 64 einen Bereich der Körperoberfläche zwischen den Elektroden trockener halten, was die elektrische Isolierung der Elektroden voneinander unterstützt. Da die wasser- bzw. feuchtigkeitsaufnehmenden Fäden 64 im Elektrodenabschnitt 30 aber von der Körperoberfläche beabstandet, also flottierend im Bereich des Tunnels 38, also auf der körperabgewandten Seite der Elektrode 20 verlaufen, führen sie dort nicht zu einer Austrocknung der Körperoberfläche im unmittelbaren Kontaktbereich zu den Elektroden 20. Auf der anderen Seite wird davon ausgegangen, dass die Wasser bzw. Feuchtigkeit aufnehmenden Fäden 64 dennoch eine gewisse Feuchtigkeitskonditionierung im Elektrodenabschnitt 30, und zwar im Bereich der Elektrode 20, ausüben können. Sie schützen die Elektrode 20 einerseits vor übermäßiger Einnässung und andererseits vor Austrocknung, indem sie ein Feuchtigkeitsreservoir in Form der in ihnen gespeicherten Feuchtigkeit zur Verfügung stellen. Dadurch, dass andererseits die elektrisch nicht leitfähigen Fäden 54 und 56 der körperabgewandten zweiten Lage 36 eine geringere Wasseraufnahmefähigkeit aufweisen als die soeben erläuterten im Elektrodenabschnitt 30 flottierenden Fäden 64, bilden diese wiederum nach außen hin einen Schutz vor Austrocknung der jeweiligen Elektrode 20 durch Verdunstung und einen Schutz vor übermäßigem Wasser- oder Feuchtigkeitseintrag von außen zu der Elektrode 20. Es wird also als besonders vorteilhaft angesehen, dass die körperabgewandte zweite Lage 36 eine äußere wasserabstoßende Abdeckung der Elektrode 20 im Elektrodenabschnitt 30 des textilen Flachmaterials 2 bildet. Insbesondere im Zusammenwirken mit auf der körperabgewandten Seite der Elektrode 20 vorgesehenen wasseranziehenden Fäden 64 lässt sich die Erzielung einer guten Kontaktierung, d.h. eines geringen Kontaktwiderstands zwischen Elektrode 20 und Hautoberfläche unterstützen. Schließlich sei noch zu dem im Zwischenabschnitt 32 des textilen Flachmaterials flottierenden Verlauf der in Längsrichtung erstreckten elektrisch leitfähigen Fäden 50 weiter ausgeführt. Wie bereits erwähnt, bilden diese Fäden 50 zusammen mit den in Querrichtung erstreckten elektrisch leitfähigen Fäden 52 die jeweilige Elektrode 20. Da die in Längsrichtung erstreckten leitfähigen Fäden 50, so wie vorzugsweise auch alle übrigen in der Längsrichtung erstreckten Fäden, bei der Fertigung des textilen Flachmaterials endlos eingebracht werden, erstrecken sie sich bei der Fertigung auch zwischen zwei Elektrodenabschnitten 30, also im Zwischenabschnitt 32. Dort erstrecken sie sich jedoch, wie schematisch in der Figur 5 und in Figur 2 links angedeutet ist, flottierend. Dies würde die aufeinanderfolgenden Elektroden 20 elektrisch leitend miteinander verbinden, was beispielsweise in Einzelfällen durchaus gewünscht sein kann. Wenn dies hingegen nicht gewünscht ist, dann besteht die Möglichkeit, diese flottierenden Fäden 50 zu schneiden, so wie dies in der Figur 2 bei Bezugszeichen 66 jeweils angedeutet ist. Auf diese Weise kann man die elektrisch leitfähigen Fäden 50 nahe dem Elektrodenabschnitt 30 schneiden und die entstehende Länge verwerfen. Es ist jedoch auch denkbar und für die Anwendung des textilen Flachmaterials 2 oder eines Flachmaterialabschnitts 16 vorteilhaft, wenn die in Längsrichtung erstreckten leitfähigen Fäden 50 nicht elektrodennah, sondern in Längsrichtung etwa mittig geschnitten werden, weil dann die jeweilige Restlängen der Fäden 50 zu einem Bündel 70 zusammengeführt werden können (in Figur 6 angedeutet), welches als Kontaktierungslitze der jeweiligen Elektrode 20 eingesetzt werden kann. Dieses Bündel 70 ist dann zudem vorteilhafterweise auf der körperabgewandten Seite des Flachmaterials 2 ergreifbar und mit von dort zu einer an sich beliebigen Mess- oder Behandlungsapparatur führenden Leitungsmitteln verbindbar (was aber nicht dargestellt ist) .

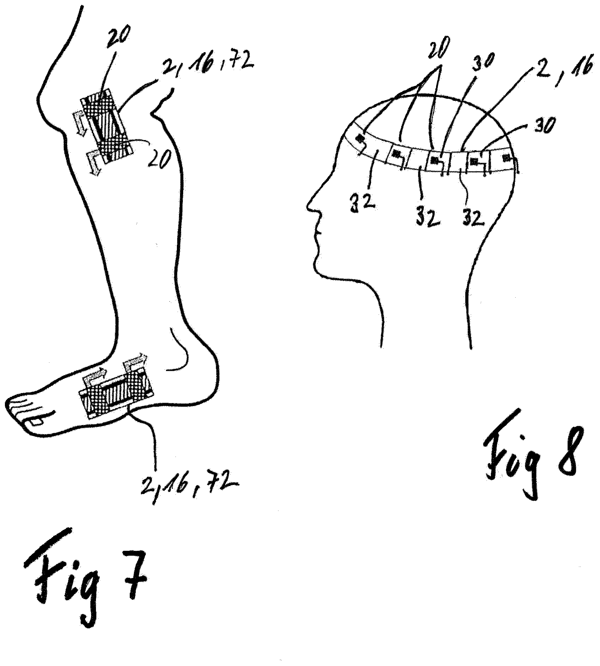

Figuren 7 und 8 zeigen rein beispielhafte Anwendungen in Form von angedeuteten Anordnungen eines insbesondere wie vorausgehend beschrieben ausgebildeten Abschnitts 16 eines erfindungsgemäßen textilen Flachmaterials 2 am menschlichen Körper. Es kann sich hierbei um einen Wundverband 72, insbesondere ein Pflaster, handeln.

Figur 8 zeigt einen in einer Kopfumfangsrichtung durchgehend erstrecktes und auf sich geschlossenes textiles Flachmaterial 2, welches also zu einem zylindrischen Abschnitt 16 zusammengefasst ist und eine Mehrzahl von Elektrodenabschnitten 30 mit Elektroden 20 und dazwischen angeordneten Zwischenabschnitten 32 umfasst.

**Patentansprüche**

1.  Textiles Flachmaterial (2) zum Anlegen an einen menschlichen oder tierischen Körper zur Erfassung und/oder Übertragung von elektrischen Signalen, umfassend:

    eine Längsrichtung (4),
    eine quer zur Längsrichtung (4) verlaufende Querrichtung (6),
    eine Erstreckungsebene (8),
    eine Dickenrichtung (10) orthogonal zur Erstreckungsebene (8),
    eine körperzugewandte erste Lage (34),
    eine körperabgewandte zweite Lage (36),
    wenigstens eine an oder in der ersten Lage (34) angeordnete Elektrode (20) zum Anlegen an den Körper, wobei die Elektrode (20) elektrisch leitfähige Fäden (50, 52) umfasst,
    wobei die zweite Lage (36) elektrisch nicht leitfähige Fäden (56, 58) mit einer ersten
    Wasseraufnahmefähigkeit umfasst, **dadurch gekennzeichnet, dass** entweder in der ersten Lage (34) oder in

EP 4 267 233 B1

Dickenrichtung (10) zwischen der Elektrode (20) und der zweiten Lage (36) in der Längsrichtung (4) sich erstreckende Fäden (64) mit einer zweiten Wasseraufnahmefähigkeit vorgesehen sind, und

dass die zweite Wasseraufnahmefähigkeit dieser Fäden (64) größer ist als die erste Wasseraufnahmefähigkeit der elektrisch nicht leitfähigen Fäden (56, 58) der zweiten Lage (36).

2. Flachmaterial nach Anspruch 1, **dadurch gekennzeichnet, dass** die zweite Wasseraufnahmefähigkeit wenigstens das 1,10-fache, insbesondere wenigstens das 2,0-fache und weiter insbesondere wenigstens das 5,0-fache, insbesondere wenigstens das 10-fache, insbesondere wenigstens das 20-fache der ersten Wasseraufnahmefähigkeit beträgt insbesondere dass die erste Wasseraufnahmefähigkeit höchstens 1,0 Gew.-%, insbesondere höchstens 0,1 Gew.-%, insbesondere höchstens 0,01 Gew.-% beträgt, und weiter insbesondere dass die zweite Wasseraufnahmefähigkeit wenigstens 2,0 Gew.-%, insbesondere wenigstens 5,0 Gew.-%, insbesondere wenigstens 20 Gew.-% beträgt.

3. Flachmaterial nach einem oder mehreren der vorstehenden Ansprüche, **dadurch gekennzeichnet, dass** die elektrisch leitfähigen Fäden (50, 52) der Elektrode (20) in der Längsrichtung (4) sich erstreckende elektrisch leitfähige Fäden (50) und in der Querrichtung (6) sich erstreckende elektrisch leitfähige Fäden (52) umfassen.

4. Flachmaterial nach einem oder mehreren der vorstehenden Ansprüche, **dadurch gekennzeichnet, dass** die elektrisch nicht leitfähigen Fäden (56, 58) der zweiten Lage (36) in der Längsrichtung (4) sich erstreckende elektrisch nicht leitfähige Fäden (56) und in der Querrichtung (6) sich erstreckende elektrisch nicht leitfähige Fäden (58) umfassen, die jeweils die erste Wasseraufnahmefähigkeit aufweisen.

5. Flachmaterial nach einem oder mehreren der vorstehenden Ansprüche, **dadurch gekennzeichnet, dass** wenigstens ein an die Elektrode (20) sich in der Querrichtung (6) anschließender Seitenbereich (26), insbesondere ein an die Elektrode (20) sich in der Querrichtung (6) anschließender erster Seitenbereich (26) und ein an die Elektrode (20) sich in der Querrichtung (6) anschließender zweiter Seitenbereich (28) gegenüber dem ersten Seitenbereich (26) vorhanden ist.

6. Flachmaterial nach einem der vorstehenden Ansprüche, **dadurch gekennzeichnet, dass** die Elektrode (20) oder die Elektrode (20) und der oder die Seitenbereiche (26, 28) einen Elektrodenabschnitt (30) des Flachmaterials (2) bilden, und dass sich an den Elektrodenabschnitt (30) in der Längsrichtung (4) ein Zwischenabschnitt (32) des Flachmaterials (2) anschließt, insbesonderedass sich ein weiterer Zwischenabschnitt (32) an den Elektrodenabschnitt (30) in der Längsrichtung (4) gegenüber dem Zwischenabschnitt (32) anschließt.

7. Flachmaterial nach einem der vorhergehenden Ansprüche, **dadurch gekennzeichnet, dass** die erste Lage (34) in dem oder den Seitenbereichen (26, 28) des Elektrodenabschnitts (30) in der Längsrichtung (4) sich erstreckende elektrisch nicht leitfähige Fäden (54) und in der Querrichtung (6) sich erstreckende elektrisch leitfähige Fäden (52) umfasst.

8. Flachmaterial nach einem oder mehreren der vorstehenden Ansprüche, **dadurch gekennzeichnet, dass** die in der Längsrichtung (4) sich erstreckenden Fäden (64) mit der zweiten Wasseraufnahmefähigkeit in der Querrichtung (6) zwischen dem ersten Seitenbereich (26) und dem zweiten Seitenbereich (28), angeordnet sind, insbesondere dass die Fäden (64) mit der zweiten Wasseraufnahmefähigkeit flottierend zwischen der Elektrode (20) und der zweiten Lage (36) vorgesehen sind.

9. Flachmaterial nach einem der vorstehenden Ansprüche, **dadurch gekennzeichnet, dass** die erste Lage (34) und die zweite Lage (36) zumindest im Elektrodenabschnitt (30) lose aneinander an- oder aufliegen.

10. Flachmaterial nach einem der Ansprüche 6 bis 9, **dadurch gekennzeichnet, dass** die erste Lage (34) und die zweite Lage (36) im Zwischenabschnitt (32) miteinander verbunden sind oder dass die erste Lage (34) und die zweite Lage (36) in dem Zwischenabschnitt (32) zur Bildung einer gemeinsamen textilen Trägerlage (42) zusammengeführt sind.

11. Flachmaterial nach einem oder mehreren der vorstehenden Ansprüche, **dadurch gekennzeichnet, dass** die Fäden (64) mit der zweiten Wasseraufnahmefähigkeit in dem Zwischenabschnitt (32) zum Anlegen an den Körper ausgebildet und angeordnet sind.

12. Flachmaterial nach einem oder mehreren der Ansprüche 6 bis 11, **dadurch gekennzeichnet, dass** die in der

9

Längsrichtung (4) angeordneten elektrisch leitfähigen Fäden (50, 52) der Elektrode (20) durch die zweite Lage (36) und/oder die Trägerlage (42) in Dickenrichtung (10) hindurchtreten und im Zwischenabschnitt (32) auf der körperabgewandten Seite der zweiten Lage (36) oder der Trägerlage (42) flottierend angeordnet sind.

13. Flachmaterial nach einem der Ansprüche 6 bis 12, **dadurch gekennzeichnet, dass** der Zwischenabschnitt (32) einen Mittenbereich (44) umfasst, wobei der Mittenbereich (44) eine im Wesentlichen der Elektrode (20) entsprechende Quererstreckung in der Querrichtung (6) aufweist, dass in dem Mittenbereich (44) in der Längsrichtung (4) sich erstreckende im Wesentlichen parallel nebeneinander angeordnete Fäden (64) mit der zweiten Wasseraufnahmefähigkeit, und in der Längsrichtung sich erstreckende und im Wesentlichen parallel nebeneinander angeordnete, elektrisch nicht leitfähige Fäden (56) mit der ersten Wasseraufnahmefähigkeit vorgesehen sind.

14. Flachmaterial nach einem der Ansprüche 6 bis 13, **dadurch gekennzeichnet, dass** die erste Lage (34) und die zweite Lage (36) derart angeordnet und miteinander verbunden sind, dass im Elektrodenabschnitt (30) ein sich in der Querrichtung (6) erstreckender Tunnel (38) gebildet ist, der durch die erste und zweite Lage (34, 36) begrenzt wird, insbesondere dass in den Tunnel (38) ein Druckelement (40) einsetzbar ist, welches die erste Lage (34) und die Elektrode und gegebenenfalls die quer durch den Tunnel flottierend geführten Fäden (64) mit der zweiten Wasseraufnahmefähigkeit zum Körper hindrängt.

15. Flachmaterial nach einem der Ansprüche 3 bis 14, **dadurch gekennzeichnet, dass** die Elektrode (20) zum Körper erhaben gegenüber den Seitenbereichen (26, 28) und/oder gegenüber dem Zwischenabschnitt (32) und/oder gegenüber der Trägerlage (42) ausgebildet ist.

16. Flachmaterial nach einem der vorhergehenden Ansprüche, **dadurch gekennzeichnet, dass** die erste und/oder die zweite Lage (34, 36) von einem Gewebe gebildet ist, wobei eine Kettrichtung des Gewebes in der Längsrichtung (4) verläuft und eine Schussrichtung des Gewebes in der Querrichtung (6) verläuft.

17. Wundverband (72) zur Unterstützung und Überwachung der Wundbehandlung, **dadurch gekennzeichnet dass** der Wundverband ein Flachmaterial (2) nach einem oder mehreren der vorstehenden Ansprüche umfasst.

## Claims

1. A flat textile material (2) for placing on a human or animal body in order to capture and/or transmit electrical signals, comprising:

   a longitudinal direction (4),
   a transverse direction (6) running transverse to the longitudinal direction (4),
   an extension plane (8),
   a thickness direction (10) orthogonal to the extension plane (8),
   a first layer (34) facing the body,
   a second layer (36) facing away from the body,
   at least one electrode (20) arranged on or in the first layer (34) for placing on the body,
   wherein the electrode (20) comprises electrically conductive threads (50, 52),
   wherein the second layer (36) comprises electrically non-conductive threads (56, 58) having a first water absorbency, **characterized in that**
   there are provided either in the first layer (34) or in the thickness direction (10) between the electrode (20) and the second layer (36) threads (64) having a second water absorbency extending in the longitudinal direction (4), and
   wherein the second water absorbency of these threads (64) is greater than the first water absorbency of the electrically non-conductive threads (56, 58) of the second layer (36).

2. The flat material as claimed in claim 1, **characterized in that** the second water absorbency is at least 1.10 times, in particular at least 2.0 times and further in particular at least 5.0 times, in particular at least 10 times, in particular at least 20 times, the first water absorbency, in particular **in that** the first water absorbency is not more than 1.0 wt.%, in particular not more than 0.1 wt.%, in particular not more than 0.01 wt.%, and further in particular **in that** the second water absorbency is at least 2.0 wt.%, in particular at least 5.0 wt.%, in particular at least 20 wt.%.

3. The flat material as claimed in one or more of the preceding claims, **characterized in that** the electrically conductive threads (50, 52) of the electrode (20) comprise electrically conductive threads (50) extending in the longitudinal direction (4) and electrically conductive threads (52) extending in the transverse direction (6).

4. The flat material as claimed in one or more of the preceding claims, **characterized in that** the electrically non-conductive threads (56, 58) of the second layer (36) comprise electrically non-conductive threads (56) extending in the longitudinal direction (4) and electrically non-conductive threads (58) extending in the transverse direction (6), which threads in each case have the first water absorbency.

5. The flat material as claimed in one or more of the preceding claims, **characterized in that** there is at least one side region (26) adjoining the electrode (20) in the transverse direction (6), in particular a first side region (26) adjoining the electrode (20) in the transverse direction (6) and a second side region (28) adjoining the electrode (20) in the transverse direction (6) opposite the first side region (26).

6. The flat material as claimed in one of the preceding claims, **characterized in that** the electrode (20) or the electrode (20) and the side region(s) (26, 28) form an electrode portion (30) of the flat material (2), and **in that** the electrode portion (30) is adjoined in the longitudinal direction (4) by an intermediate portion (32) of the flat material (2), in particular **in that** the electrode portion (30) is adjoined in the longitudinal direction (4) by a further intermediate portion (32) opposite the intermediate portion (32).

7. The flat material as claimed in one of the preceding claims, **characterized in that** the first layer (34) comprises in the side region(s) (26, 28) of the electrode portion (30) electrically non-conductive threads (54) extending in the longitudinal direction (4) and electrically conductive threads (52) extending in the transverse direction (6).

8. The flat material as claimed in one or more of the preceding claims, **characterized in that** the threads (64) having the second water absorbency extending in the longitudinal direction (4) are arranged in the transverse direction (6) between the first side region (26) and the second side region (28), in particular **in that** the threads (64) having the second absorbency are provided in a floating manner between the electrode (20) and the second layer (36).

9. The flat material as claimed in one of the preceding claims, **characterized in that** the first layer (34) and the second layer (36) lie loosely against or on one another at least in the electrode portion (30).

10. The flat material as claimed in one of claims 6 to 9, **characterized in that** the first layer (34) and the second layer (36) are joined together in the intermediate portion (32) or **in that** the first layer (34) and the second layer (36) are brought together in the intermediate portion (32) to form a combined textile carrier layer (42).

11. The flat material as claimed in one or more of the preceding claims, **characterized in that** the threads (64) having the second water absorbency in the intermediate portion (32) are configured and arranged to be placed on the body.

12. The flat material as claimed in one or more of claims 6 to 11, **characterized in that** the electrically conductive threads (50, 52) of the electrode (20) that are arranged in the longitudinal direction (4) pass through the second layer (36) and/or the carrier layer (42) in the thickness direction (10) and are arranged in the intermediate portion (32) in a floating manner on the side of the second layer (36) or of the carrier layer (42) facing away from the body.

13. The flat material as claimed in one of claims 6 to 12, **characterized in that** the intermediate portion (32) comprises a middle region (44), wherein the middle region (44) has a transverse extent in the transverse direction (6) that corresponds substantially to the electrode (20), **in that** there are provided in the middle region (44) threads (64) having the second water absorbency which extend in the longitudinal direction (4) and are arranged side by side substantially in parallel, and electrically non-conductive threads (56) having the first water absorbency which extend in the longitudinal direction and are arranged side by side substantially in parallel.

14. The flat material as claimed in one of claims 6 to 13, **characterized in that** the first layer (34) and the second layer (36) are arranged and joined together such that there is formed in the electrode portion (30) a tunnel (38) which extends in the transverse direction (6) and is delimited by the first and second layers (34, 36), in particular **in that** a pressure element (40) can be inserted into the tunnel (38), which pressure element urges the first layer (34) and the electrode, and optionally the threads (64) having the second water absorbency which are guided transversely through the tunnel in a floating manner, toward the body.

15. The flat material as claimed in one of claims 3 to 14, **characterized in that** the electrode (20) is configured to be raised relative to the body as compared to the side regions (26, 28) and/or as compared to the intermediate portion (32) and/or as compared to the carrier layer (42).

**16.** The flat material as claimed in one of the preceding claims, **characterized in that** the first and/or the second layer (34, 36) is formed by a woven fabric, wherein a warp direction of the woven fabric runs in the longitudinal direction (4) and a weft direction of the woven fabric runs in the transverse direction (6).

**17.** A wound dressing (72) for assisting and monitoring wound treatment, **characterized in that** the wound dressing comprises a flat material (2) as claimed in one or more of the preceding claims.

**Revendications**

**1.** Matériau plat textile (2) destiné à être appliqué sur un corps humain ou animal et à détecter et/ou à transmettre des signaux électriques, comprenant:

une direction longitudinale (4),
une direction transversale (6) s'étendant transversalement à la direction longitudinale (4),
un plan d'extension (8),
une direction d'épaisseur (10) orthogonale au plan d'extension (8),
une première couche (34) montrant vers le corps,
une deuxième couche (36) montrant dans la direction opposée au corps,
au moins une électrode (20) qui est disposée sur ou dans la première couche (34) et destinée à être appliquée sur le corps,
dans lequel l'électrode (20) comprend des fils électriquement conducteurs (50, 52),
dans lequel la deuxième couche (36) comprend des fils électriquement non conducteurs (56, 58) présentant une première capacité d'absorption d'eau, **caractérisé par le fait que** des fils (64) s'étendant dans la direction longitudinale (4) et présentant une deuxième capacité d'absorption d'eau sont prévus soit dans la première couche (34) soit dans la direction d'épaisseur (10) entre l'électrode (20) et la deuxième couche (36), et que la deuxième capacité d'absorption d'eau de ces fils (64) est supérieure à la première capacité d'absorption d'eau des fils électriquement non conducteurs (56, 58) de la deuxième couche (36).

**2.** Matériau plat selon la revendication 1, **caractérisé par le fait que** la deuxième capacité d'absorption d'eau est d'au moins 1,10 fois, en particulier d'au moins 2,0 fois et en outre en particulier d'au moins 5,0 fois, en particulier d'au moins 10 fois, en particulier d'au moins 20 fois la première capacité d'absorption d'eau, en particulier que la première capacité d'absorption d'eau est de 1,0 % en poids tout au plus, en particulier de 0,1 % en poids tout au plus, en particulier de 0,01 % en poids tout au plus, et en outre en particulier que la deuxième capacité d'absorption d'eau est d'au moins 2,0 % en poids, en particulier d'au moins 5,0 % en poids, en particulier d'au moins 20 % en poids.

**3.** Matériau plat selon une ou plusieurs des revendications précédentes, **caractérisé par le fait que** les fils électriquement conducteurs (50, 52) de l'électrode (20) sont des fils électriquement conducteurs (50) s'étendant dans la direction longitudinale (4) et des fils électriquement conducteurs (52) s'étendant dans la direction transversale (6).

**4.** Matériau plat selon une ou plusieurs des revendications précédentes, **caractérisé par le fait que** les fils électriquement non conducteurs (56, 58) de la deuxième couche (36) sont des fils électriquement non conducteurs (56) s'étendant dans la direction longitudinale (4) et des fils électriquement non conducteurs (58) s'étendant dans la direction transversale (6) qui présentent chacun la première capacité d'absorption d'eau.

**5.** Matériau plat selon une ou plusieurs des revendications précédentes, **caractérisé par le fait qu'**il existe au moins une zone latérale (26) qui est contiguë à l'électrode (20) dans la direction transversale (6), en particulier une première zone latérale (26) qui est contiguë à l'électrode (20) dans la direction transversale (6) et une deuxième zone latérale (28) opposée à la première zone latérale (26), qui est contiguë à l'électrode (20) dans la direction transversale (6).

**6.** Matériau plat selon l'une quelconque des revendications précédentes, **caractérisé par le fait que** l'électrode (20) ou l'électrode (20) et la ou les zones latérales (26, 28) forment une section d'électrode (30) du matériau plat (2), et qu'une section intermédiaire (32) du matériau plat (2) est contiguë à la section d'électrode (30) dans la direction longitudinale (4), en particulier qu'une autre section intermédiaire (32) est contiguë à la section d'électrode (30) dans la direction longitudinale (4) de manière opposée à la section intermédiaire (32).

7. Matériau plat selon l'une quelconque des revendications précédentes, **caractérisé par le fait que** la première couche (34) dans la ou les zones latérales (26, 28) de la section d'électrode (30) comprend des fils électriquement non conducteurs (54) s'étendant dans la direction longitudinale (4) et des fils électriquement conducteurs (52) s'étendant dans la direction transversale (6).

8. Matériau plat selon une ou plusieurs des revendications précédentes, **caractérisé par le fait que** les fils (64) s'étendant dans la direction longitudinale (4) et présentant la deuxième capacité d'absorption d'eau sont disposés dans la direction transversale (6) entre la première zone latérale (26) et la deuxième zone latérale (28), en particulier que les fils (64) présentant la deuxième capacité d'absorption d'eau sont prévus de manière flottante entre l'électrode (20) et la deuxième couche (36).

9. Matériau plat selon l'une quelconque des revendications précédentes, **caractérisé par le fait que** la première couche (34) et la deuxième couche (36) s'appliquent de manière lâche l'une sur ou contre l'autre au moins dans la section d'électrode (30).

10. Matériau plat selon l'une quelconque des revendications 6 à 9, **caractérisé par le fait que** la première couche (34) et la deuxième couche (36) sont reliées l'une à l'autre dans la section intermédiaire (32) ou que la première couche (34) et la deuxième couche (36) sont réunies dans la section intermédiaire (32) pour former une couche de support textile commune (42).

11. Matériau plat selon une ou plusieurs des revendications précédentes, **caractérisé par le fait que** les fils (64) présentant la deuxième capacité d'absorption d'eau sont conçus et disposés dans la section intermédiaire (32) destinée à être appliquée sur le corps.

12. Matériau plat selon une ou plusieurs des revendications 6 à 11, **caractérisé par le fait que** les fils électriquement conducteurs (50, 52) de l'électrode (20) qui sont disposés dans la direction longitudinale (4) passent dans la direction d'épaisseur (10) à travers la deuxième couche (36) et/ou la couche de support (42) et sont disposés de manière flottante dans la section intermédiaire (32) du côté de la deuxième couche (36) ou de la couche de support (42), qui montre dans la direction opposée au corps.

13. Matériau plat selon l'une quelconque des revendications 6 à 12, **caractérisé par le fait que** la section intermédiaire (32) comprend une zone centrale (44), dans lequel la zone centrale (44) présente une extension transversale dans la direction transversale (6), qui correspond pour l'essentiel à l'électrode (20), que dans la zone centrale (44) sont prévus des fils (64) qui s'étendent dans la direction longitudinale (4), sont disposés pour l'essentiel parallèlement les uns à côté des autres et présentent la deuxième capacité d'absorption d'eau, et des fils électriquement non conducteurs (56) qui s'étendent dans la direction longitudinale, sont disposés pour l'essentiel parallèlement les uns à côté des autres et présentent la première capacité d'absorption d'eau.

14. Matériau plat selon l'une quelconque des revendications 6 à 13, **caractérisé par le fait que** la première couche (34) et la deuxième couche (36) sont disposées et reliées l'une à l'autre de telle sorte que dans la section d'électrode (30) est formé un tunnel (38) qui s'étend dans la direction transversale (6) et qui est délimité par les première et deuxième couches (34, 36), en particulier qu'un élément de pression (40) peut être inséré dans le tunnel (38), qui pousse vers le corps la première couche (34) et l'électrode et, le cas échéant, les fils (64) guidés de manière flottante à travers le tunnel et présentant la deuxième capacité d'absorption d'eau.

15. Matériau plat selon l'une quelconque des revendications 3 à 14, **caractérisé par le fait que** l'électrode (20) vers le corps est conçue de manière à être surélevée par rapport aux zones latérales (26, 28) et/ou par rapport à la section intermédiaire (32) et/ou par rapport à la couche de support (42).

16. Matériau plat selon l'une quelconque des revendications précédentes, **caractérisé par le fait que** la première et/ou la deuxième couche (34, 36) est formée par un tissu, dans lequel une direction chaîne du tissu s'étend dans la direction longitudinale (4) et une direction trame du tissu s'étend dans la direction transversale (6).

17. Pansement (72) destiné à soutenir et à surveiller le traitement d'une plaie, **caractérisé par le fait que** le pansement comprend un matériau plat (2) selon une ou plusieurs des revendications précédentes.

Fig. 1

Fig. 2

Fig 3

Fig 4a

Fig 4b

34

56

42

36

18

50

58

68

Fig 5

Fig 6

Fig 7

Fig 8

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102017126463 A1

- EP 3169219 B1